# FASCICULE DE BREVET EUROPEEN

(11) **EP 0 979 301 B1**
(45) Date de publication et mention de la délivrance du brevet: **08.09.2004**
(21) Numéro de dépôt: 98913880.5
(22) Date de dépôt: 11.03.1998
(51) Int. Cl.: C12P 19/04, C08B 37/04, C12N 9/88

(54) **SOUCHE DE PSEUDOMONAS ALGINOVORA PRODUCTRICE D'ALGINATE-LYASE ET SON UTILISATION POUR LA DEPOLYMERISATION D'ALGINATE**
ALGINAT-LYASE PRODUZIERENDER PSEUDOMONAS ALGINOVORA STAMM UND SEINE VERWENDUNG ZUR DEPOLYMERISATION VON ALGINAT
PSEUDOMONAS ALGINOVORA STRAIN PRODUCING ALGINATE-LYASE AND ITS USE FOR THE DEPOLYMERISATION OF ALGINATE

(30) Priorité: 11.03.1997 FR 9703218
(43) Date de publication de la demande: 16.02.2000
(73) Titulaire: IFREMER INSTITUT FRANCAIS DE RECHERCHE POUR L'EXPLOITATION DE LA MER, 92130 Issy-les-Moulineaux (FR)
(72) Inventeur: BRAULT, Dominique, F-22610 Pleubian (FR); COLIN, Anne, F-22610 L'Armor Pleubian (FR); HERAULD, Alain, F-38113 Veuret Voroize (FR); JOYET, Valérie, F-22500 Paimpol (FR); LOGNONE, Vincent, F-22610 Pleubian (FR); MARCHAL, Pascal, F-22500 Paimpol (FR); DURAND, Patrick, F-44400 Reze (FR)
(74) Mandataire: Branger, Jean-Yves
(86) Numéro de dépôt international: PCT/FR1998/000483
(87) Numéro de publication internationale: WO 1998/040511

(56) Documents cités:
- FR-A- 2 690 445
- US-A- 5 348 875
- CHAVAGNAT, FREDERIC ET AL: "Cloning, sequencing and overexpression in Escherichia coli of the alginate lyase -encoding aly gene of Pseudomonas alginovora: identification of three classes of alginate lyases" BIOCHEM. J. (1996), 319(2), 575-583 CODEN: BIJOAK;ISSN: 0264-6021, 1996, XP002046907 cité dans la demande
- C. BOYEN ET AL.: "Preparation of guluronate lyase from Pseudomonas alginovora for protoplast isolation in Laminaria" ENZYME AND MICROBIAL TECHNOLOGY, vol. 12, no. 11, novembre 1990, pages 885-890, XP002046908 cité dans la demande
- SHINICHI KINOSHITA ET AL.: "Isolation of an alginate-degrading organism and purification of its alginate lyase" JOURNAL OF FERMENTATION AND BIOENGINEERING, vol. 72, no. 2, 1991, pages 74-78, XP000673181

## Description

La présente invention concerne une nouvelle souche bactérienne de Pseudomonas alginovora et son utilisation pour la production d'alginate-lyase.

Elle se rapporte également notamment à un procédé de dépolymérisation d'alginate et aux fragments d' alginate obtenus conformément à ce procédé.

L'alginate est un polysaccharide présent notamment dans les algues brunes et dans certaines bactéries.

Il s'agit d'une famille de composés linéaires constitués de copolymères d' acide β-D-mannuronique et d' acide α-L-guluronique liés en 1-4.

Il est communément admis que la molécule d'alginate se présente sous la forme de blocs homogènes polymannuronates (poly M), polyguluronates (poly G), et/ou de blocs mixtes (poly MG).

Du fait de ses propriétés intéressantes, à savoir épaississante, stabilisante et gélifiante, l'alginate a trouvé des applications dans de nombreux domaines dont, notamment, le secteur agro-alimentaire.

De plus, de nombreuses alginases, enzymes présentant la spécificité de dégrader l'alginate ont été décrites et isolées à partir de mollusques marins, de bactéries ou d'algues. Elles sont de type alginate-lyase, ce qui signifie qu'elles dépolymérisent l'alginate par une réaction de β-élimination. En d'autres termes, la réaction produit un résidu insaturé à l'extrémité non-réductrice de la liaison coupée.

Ces alginate-lyases peuvent donc être utilisées pour l'élimination d'alginate employé de façon transitoire dans des procédés industriels, notamment dans l'industrie textile.

En effet, les alginates sont utilisés comme épaississants des pâtes d'impression ou comme agents d' encollage de fils avant tissage, ou encore comme agent d'apprêt pour la finition des tissus. Après tissage, le tissu doit subir un désencollage pour éliminer les alginates, qui peut être réalisé par traitement avec des alginate-lyases.

Une autre application potentielle des alginate-lyases est celle dans laquelle on recherche à éliminer les alginates utilisés comme épaississants dans des formulations de colorants pour tissus.

Encore une autre application potentielle est celle de la lutte contre le "fouling" dans des circuits de refroidissement, notamment de centrales nucléaires, c'est-à-dire contre le développement de populations bactériennes et micro-algales.

Enfin, une autre application de ces alginate-lyases consiste à transformer les alginates de haut poids moléculaire en polysaccharides de degré de polymérisation inférieur, jusqu'à obtention d'oligosaccharides spécifiques.

Par oligosaccharides, on entend des saccharides d'unités monomères M et/ou G, dont le degré de polymérisation est compris entre 2 et 20.

En effet, des travaux ont montré que certaines de ces fractions oligo alginiques avaient des effets bénéfiques sur la flore intestinale, via un effet "bifidogène" semblable à celui qui est déjà connu pour les oligofructosaccharides. En outre, certaines fractions d'oligosaccharides d'alginate ont des effets éliciteurs qui présentent un intérêt dans le domaine de la protection des végétaux.

Par ailleurs, des oligosaccharides peuvent également être utilisés pour être introduits dans des préparations cosmétiques.

Cependant, quelle que soit l'application envisagée, on se heurte à la spécificité des alginate-lyases. Certaines, de type guluronate-lyases, s'avèrent incapables de dégrader les segments poly M. D' autres, de type mannuronate-lyases sont incapables de dégrader les segments poly G. Par conséquent, dès lors que l'on souhaite obtenir les deux activités, par exemple pour réduire le degré de polymérisation moyen du milieu dans lequel est présent l' alginate, il est nécessaire d'utiliser les deux types d'enzyme.

On comprend que ceci entraîne inévitablement une complication du processus de dépolymérisation de l'alginate et, bien entendu un surcoût.

Ainsi, les documents C. BOYEN et al : "Preparation of guluronate-lyase from Pseudomonas alginovora for protoplast isolation in Laminaria" ENZYME AND MICROBIAL TECHNOLOGY, vol. 12, no. 11, novembre 1990, pages 885-890, et CHAVAGNAT, Frederic et al : "Cloning, sequencing and overexpression in Escherichia coli of the aglinate-lyase - encoding aly gene of Pseudomonas alginovora : identification of three classes of alginate lyases" BIOCHEM. J. (1996), 319(2), 575-583, se rapportent à la production d'alginate-lyase à partir d'une souche bactérienne de Pseudomonas alginovora déposé à l'Institut Pasteur sous le n° CIP 102 941.

Le document BOYEN décrit l'alginate-lyase comme ayant une activité guluronate-lyase stricte. Elle est présentée comme étant constitutive, mais il est précisé que les performances optimales de l'enzyme sont obtenues par fermentation sur un substrat contenant de l'alginate.

Le document CHAVAGNAT démontre, par une technique de clonage, la présence d' une activité stricte mannuronate-lyase sur l'enzyme obtenue, résultats qui vont à l'encontre de ceux décrits dans le document BOYEN. Il est précisé que l'enzyme "aurait" en plus une activité guluronate-lyase, sans que cette activité spécifique soit démontrée.

Deux activités distinctes sont donc décrites par BOYEN et CHAVAGNAT, mais pour des alginate-lyases obtenues dans des conditions différentes.

La présente invention a justement pour but de pallier ces inconvénients.

Ainsi, l'invention fournit une nouvelle souche bactérienne de Pseudomonas alginovora qui permet d'obtenir une alginate-lyase à double activité, à savoir mannuronate- et guluronate-lyase.

Elle fournit également un procédé de dépolymérisation d'alginate, qui permet d'obtenir aussi bien des fractions formées strictement d'unités mannuronate ou guluronate que des fractions mixtes.

La nouvelle souche bactérienne dont il s'agit a été déposée le 6 mars 1998 auprès de la C.N.C.M. sous le numéro I - 1989.

L'invention concerne également l'utilisation d'une telle souche pour la production d'une alginate-lyase à activités mannuronate- et guluronate-lyase, ainsi que l'alginate-lyase obtenue par la mise en culture de la souche telle que définie plus haut.

L'invention a aussi pour objet un procédé de préparation d'alginate-lyase qui se caractérise par le fait que l'on met en culture la souche telle que définie plus haut, dans un milieu qui comprend une source d' azote, une source de carbone, un extrait de levure, de l'eau de mer et un agent anti-mousse, et que l'on récupère l'alginate-lyase obtenue.

Selon des caractéristiques non limitatives de ce procédé :
- la source d'azote consiste en de la protéine de pommes de terre ;
- la source de carbone consiste en du dextrose monohydraté ;
- l'eau de mer est filtrée à un micromètre ;
- l'agent anti-mousse est de l'huile de soja ;
- le pH du milieu est ajusté à 8,2.

Le procédé de dépolymérisation d'alginate selon l'invention consiste à mettre de l'alginate à incuber dans une solution contenant au moins un sel mono- ou divalent à une force ionique au moins égale à 0,05 M, en présence d'une alginate-lyase produite à partir de la souche bactérienne précitée, et, éventuellement, à récupérer la fraction de fragments d' alginate dépolymérisé obtenus.

Par ailleurs, selon d'autres caractéristiques avantageuses mais non limitatives de ce procède :
- le pH de la solution est de l'ordre de 7,5 ;
- l' alginate-lyase se présente sous forme sèche ou liquide et dans ce dernier cas, ledit liquide consiste en l'enzyme solubilisée dans une solution tampon à pH 7,5 de trishydroxyméthylaminométhane (2-amino-2-hydroxyméthyl-1,3-propanediol) et de chlorure de sodium ;
- on utilise de 0,1 à 5 % en poids d'enzyme par rapport au poids d'alginate ;
- on laisse incuber pendant 2 à 20 heures ;
- on laisse incuber pendant 8 heures environ ;
- la solution contenant au moins un sel mono- ou divalent est tamponnée, par exemple avec du trishydroxyméthylaminométhane ;
- le sel consiste en un chlorure ou en un sulfate :

- le chlorure consiste en un chlorure de sodium ou en un chlorure de magnésium hexahydraté ;
- le procédé est mis en oeuvre dans le cadre de la production d' oligosaccharides ;
- le procédé est mis en oeuvre dans le cadre de la réduction de la viscosité d'un milieu contenant de l'alginate ;
- le procédé est mis en oeuvre dans le cadre de l'extraction des fucanes d'algues brunes.

L'invention concerne également les fragments d'alginate dépolymérisé obtenus conformément à ce procédé.

Le demandeur a donc mis en évidence qu'en soumettant un alginate à l'action d'une alginate-lyase particulière dans des conditions d'incubation spécifiques, on arrive à obtenir des fragments de masses moléculaires variées, l'alginate-lyase ayant une action M-lyase, ou M-lyase et G-lyase.

L'alginate-lyase dont il s'agit est celle obtenue à partir de Pseudomonas alginovora.

Cette lyase est obtenue à partir de la souche de Pseudomonas alginovora déposée à la Collection Nationale de Culture de Microorganismes (CNCM) de l'Institut Pasteur à Paris le 6 mars 1998 sous la référence d'identification CEVA-01-98 et sous le numéro d'enregistrement I-1989.

Cette souche est une souche mutante de celle décrite dans le document BOYEN précité, laquelle a été repiquée en milieu de culture de nombreuses fois.

Une telle alginate-lyase présente les paramètres suivants :
- Présentation : sous forme sèche, poudre de couleur crème ;
- Activité: 1000 000 U/g ;
- Qualité microbiologique : FT < 10 000 germes/g levures et moisissures < 10 germes/g ;
- Stabilité : supérieure à 6 mois à - 20°C ;
- Stabilité après solubilisation : supérieure à 7 jours à 22°C ;
- Conditions d'utilisation: pH : 6,5 à 8,5 ; température : ambiante ; force ionique : 0,1 à 0,5 M.

L'activité alginate-lyase est mesurée par l'augmentation de la densité optique à 235 nm grâce à la conjugaison entre la double liaison (formée lors de la réaction de β-élimination) et le groupement carboxylique.

De façon pratique, l'activité alginate lyase est mesurée en incubant 10 à 100 microlitres de préparation enzymatique dans deux millilitres de solution de substrat (à 22°C et à pH 7,5) contenant :
- de l'alginate de sodium à 0,5 % (poids/volume) ;
- du chlorure de magnésium hexahydraté à 6.67.10-2 moles/litre (soit une force ionique égale à 0,2 moles/litre) ;
- du trishydroxyméthylaminométhane : 20.10⁻³ moles/litre.

La préparation enzymatique est réalisée en solubilisant l'enzyme sous forme sèche à 10 grammes/litre dans une solution tampon (trishydroxyméthylaminométhane : 20.10⁻³ moles/litre, chlorure de sodium 2 moles/litre, pH 7,5).

L'unité d'activité arbitraire notée UA correspond à l'augmentation d'une unité de densité optique à 235 nanomètres en une minute dans le milieu réactionnel. Le coefficient d'extinction molaire des acides uroniques insaturés étant de 5 050 litre/mole/centimètre, l'unité arbitraire (UA) équivaut à 396.10-9 moles de coupures/minute. L'unité alginate lyase (notée U) est exprimée en nanomoles de coupures/minute (1U = 10⁻⁹ moles de coupures/minute).

Comme indiqué plus haut, la production d'alginate-lyases (EC 4.2.2.3.) préférée est obtenue par culture immergée en batch de Pseudomonas alginovora n°1 - 1989.

Le milieu de culture de l'alginate-lyase comprend :
- une source d'azote : protéine de pommes de terre ;
- une source de carbone : dextrose monohydraté standard ;
- un extrait de levure ;
- de l'eau de mer filtrée à 1 micromètre ;
- un antimousse : huile de soja.

Le pH est ajusté à 8,2 avant l'autoclavage.

Le fait que l'alginate ne soit pas indispensable pour la production de l'enzyme et puisse être remplacé par une autre source de carbone indique que l' alginate-lyase est constitutive.

La production d'alginate-lyase est effectuée en fermenteur (de 2 à 20 litres), l'inoculum représentant 1 à 5 % du volume du fermenteur.

L'arrêt de la fermentation est réalisé après 10 à 20 heures de croissance, selon la taille de l' inoculum.

Pour procéder à la récupération de l' enzyme, on procède en trois étapes :
- étape 1 : centrifugation et microfiltration sur filtres à 1,6 micromètres, ou microfiltration à 0,45 micromètres ; à ce stade, une microfiltration à 0,2 microns peut être réalisée (non obligatoire) ;
- étape 2 : concentration 10 fois du volume de fermentation par ultrafiltration sur membrane à 8 000 ou 10 000 Dalton ;
- étape 3 : lyophilisation.

Le produit enzymatique final est conservé à -20°C.

### I - Production de fragments d'alginate utilisables comme oligosaccharides :

### Exemple 1

1,25 grammes d'alginate de sodium de Laminaria digitata sont placés dans 250 millilitres de solution à pH 7,5 contenant 20.10-3 moles/litre de tampon trishydroxyméthylaminométhane et 6,67.10-2 moles/litre de chlorure de magnésium hexahydraté, ce qui correspond à une force ionique de 0,2 M.

Cette préparation est mise à incuber dans 6,25 millilitres de préparation enzymatique. Cette dernière contient 5% en poids d'enzyme par rapport au poids d'alginate, soit 62,5mg (ou 66 000 U).

La préparation contient une solution tampon à pH 7,5 contenant 20.10⁻³ moles/litre de trishydroxyméthylaminométhane et 2 moles/litres de chlorure de sodium.

Bien entendu, le sel contenu dans la préparation liquide d'enzyme n'est pas forcément le même que celui ajouté à l'alginate. Par ailleurs, des sels mono- ou divalents, autres que les chlorures, peuvent être employés. Il peut s'agir par exemple de sulfates.

L'incubation enzymatique est réalisée à 25°C sous agitation magnétique avant d'être arrêtée par baisse de pH à 6,5 et traitement thermique une heure à 100°C. Le produit obtenu est ensuite centrifugé à 10 000 g pendant 30 minutes à température ambiante avant d'être filtré sur tamis à 0,22 µm.

Un premier fractionnement est réalisé sur une colonne de chromatographie d'exclusion stérique en polyacrylamide (45-90 micromètres) (colonne 1 mètre x 4,5 centimètres). Les fragments d'alginate sont élues isocratiquement avec du nitrate de sodium (5.10⁻² moles/litre) et de l'azoture de sodium (0,2 %). Le système comprend, en outre, une pompe délivrant un débit de 90 millilitres/heure, un détecteur réfractométrique et un enregistreur. Les fractions sont récupérées avec un collecteur.

### Exemple 2

On répète les étapes décrites à l'exemple 1, mais en utilisant cette fois-ci 1 % en poids d'enzyme par rapport au poids d'alginate (qui reste inchangé), soit 12,5 milligrammes (13 200 U).

### Exemple 3

On répète à nouveau les étapes de l'exemple 1, mais en utilisant 0,1 % en poids d'enzyme par rapport au poids d'alginate (toujours inchangé), soit 1,25 milligrammes (1320 U).

### Analyse des produits obtenus conformément aux exemples 1 à 3

Une étude de l'évolution des degrés de polymérisation moyen des produits formés en fonction de la quantité d'enzyme ajoutée et du temps d'incubation a été réalisée.

Comme cela est bien connu, le degré de polymérisation moyen peut se calculer à partir de la densité optique du produit à 235 nanomètres. Une unité de densité optique équivaut à la présence de 1,98 x 10-4 moles/libre de produits insaturés. Le rapport concentration initiale en alginate/concentration en produits insaturés correspond au degré de polymérisation moyen.

Cette étude montre que quelle que soit la concentration de l'enzyme ajoutée, le degré de polymérisation chute rapidement, ce qui confirme le comportement endolytique de l'enzyme.

A 5 % d'enzyme et au terme de 2 heures d' incubation, l'ensemble des polysaccharides est dégradé et les fragments produits sont, au plus de degré 10 (degré de polymérisation moyen). Ces degrés de polymérisation sont inférieurs et égaux à 6 et 5 respectivement au terme de 8 et 20 heures d'incubation. En pratique, la réaction enzymatique peut être considérée comme globalement terminée au terme de 8 heures d' incubation, les fragments produits étant majoritairement de degré de polymérisation 3 et 4.

A 1 % d'enzyme, une dégradation d'une heure est insuffisante pour dépolymériser l'ensemble des hautes masses. Une incubation de 20 heures est nécessaire pour produire des fragments de degré de polymérisation moyen de l'ordre de 4.

### II - Nature des fragments produits :

On notera que les fragments étudiés ci-dessous sont ceux produits par incubation à 5 % d' enzyme.

Un deuxième fractionnement est nécessaire car chaque fraction correspond à un mélange de fragments.

Les fractions collectées lors du premier fractionnement sont d'abord dessalées sur une colonne en polymère d'éthylèneglycol et de méthacrylate (30-60 micromètres) avant d'être purifiées sur une colonne échangeuse d'anions semi-préparative (colonne chromatographique constituée d'une phase ammonium quaternaire sur silice (5 micromètres)).

L'élution est isocratique (nitrate de sodium, débit de la pompe : 0,8 millilitres/heure), la concentration de l' éluant étant de 0,12 moles/litre pour les fragments de degré de polymérisation 4 et 5.

Les fractions sont à nouveau collectées et dessalées. Les fragments "purifiés" sont analysés :
- en chromatographie liquide haute performance (colonne chromatographique constituée d'une phase ammonium quaternaire sur silice (5 micromètres), éluant nitrate de sodium 0,12 moles/litre pour les oligosaccharides de degré de polymérisation 2 et 3, 0,15 moles/litre pour les oligosaccharides de degré de polymérisation 4 et 5) ;
- en résonance magnétique nucléaire.

La structure des fragments d'alginate a pu être déterminée de façon relativement précise jusqu'à des fragments de degré de polymérisation 4. Les molécules sont données selon le modèle ΔX où :
- Δ représente l'unité possédant une fonction insaturée, qui est la même, que l'alginate-lyase ait coupé un bloc poly M, poly G ou poly MG. Elle présente la formule suivante : 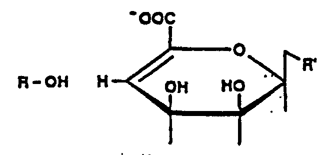 qui résulte de l'action de l'alginate-lyase sur les blocs M et G suivants : 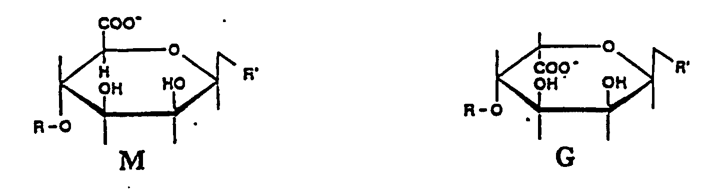 ΔX étant l'acide 4-désoxy-L-érythro-5-hexose-ulase-uronique.
- X représente une unité uronique (X = M pour l'acide mannuronique, X = G pour l'acide guluronique). L'unité uronique se trouvant à droite de la molécule possède une fonction réductrice.

Le degré de polymérisation de la molécule représentée ΔX est ici de 2.

Les structures des fragments produits mises en évidence sont données dans le tableau ci-dessous.

| Degré de polymérisation | Structure mise en évidence |
|---|---|
| 2 | ΔM ΔG |
| 3 | ΔGG ΔMM ΔMG |
| 4 | ΔGGG ΔMGG ΔGGM ΔMGM ΔMMM |

### III - Mode de fonctionnement de l'enzyme :

La mise en évidence d'oligosaccharides de type ΔGG, ΔGGG, ΔMM, ΔMGM et ΔMMM révèle que l'enzyme AL 951 utilisée en présence de sel(s) mono- ou divalent(s) coupe à la fois les blocs polymannuronates et polyguluronates, y compris les blocs mixtes. Deux activités sont donc présentes : une activité M-lyase (rupture de la liaison M-M) et une activité G-lyase (rupture de la liaison G-G).

### IV - Utilisation de l'alginate-lyase pour réduire la viscosité d'une solution d'alginate:

L'alginate-lyase peut être mise en oeuvre comme auxiliaire technologique pour abaisser rapidement la viscosité d'extraits contenant de l' alginate.

Ainsi, des tests sont réalisés sur un alginate de sodium solubilisé à 0,4 % (poids/volume) dans une solution tampon à pH 7,5 contenant du trishydroxyméthylaminométhane à 10.10-3 moles/litre, et du chlorure de sodium à 0,1 mole/litre. L'enzyme est additionnée à trois teneurs différentes (0,1 ; 1 ; 10 % poids/poids).

La baisse de viscosité est suivie directement dans le corps de mesure d'un viscosimètre selon un programme de mesure de la viscosité en fonction du temps à un gradient de vitesse égal à 200 s⁻¹. La température est maintenue à 22°C.

Les résultats montrent, que pour les trois essais, la chute de viscosité est rapide (abaissement de 30 à 80 % de la viscosité initiale apparente en quelques minutes), ce qui confirme bien que l'alginate-lyase est une endolyase (coupe au sein de la molécule d'alginate).

### V - Utilisation de l'alginate-lyase comme auxiliaire industriel pour l'extraction des fucanes :

L' alginate-lyase constitue un outil technologique intéressant pour améliorer les rendements d'extraction des fucanes, polysaccharides pariétaux complexes des algues brunes présentant des propriétés anticoagulantes et antithrombiques.

Les procédés d'extraction par voie aqueuse déjà connus aboutissent à des rendements faibles (2 à 3 % de la matière algale) alors que les teneurs en fucanes varient entre 15 et 25 % de la masse de l'algue. Pour améliorer les rendements d'extraction, l'idée est de solubiliser les alginates contenus dans les parois pour extraire conjointement les fucanes.

La mise en oeuvre de l'alginate-lyase selon le procédé conforme à l' invention présente un double intérêt :
- réduction de la viscosité de la solution et donc traitement ultérieur de l' extrait techniquement facilité ;
- amélioration des rendements d' extraction.

On obtient ainsi des rendements de 15 à 24 % selon des essais, ce qui améliore significativement les résultats obtenus par la voie classique.

La méthode d'extraction comporte différentes étapes :
1) Fabrication d'une farine finement broyée à partir d'Ascophyllum nodosum ;
2) Mise en solution de la farine à 5 % ;
3) Solubilisation des alginates par :
   - lixiviation
      Cette étape consiste à faire macérer les algues en milieu acide afin de transformer les alginates d'ions divalents en acide alginique. Un mélange à 5 % en algues est réalisé à partir de la farine placée dans une solution d'acide sulfurique à 0,1 mole/litre pendant 2 heures sous agitation.
   - Ajout d'un séquestrant des ions calcium et ajustement de pH à 7,5.
      17 % d'acide citrique (par rapport à la masse d'algues sèches) sont ajoutés au milieu acide. Le pH de la suspension est ajusté à pH 7,5 par ajout d'une solution d' hydroxyde de sodium. L'alginate passe alors sous une forme soluble. Les ions calcium sont séquestrés par le citrate de sodium.
4) Dépolymérisation des alginates :
   L'enzyme sous forme lyophilisée est préalablement solubilisée dans un tampon (trishydrométhylaminométhane à 20.10⁻³ moles/litre, chlorure de sodium à 2 moles/litre, pH 7,5) puis est additionnée à raison d'un rapport enzyme/algue variant de 1 à 5 %. La réaction se déroule à pH 7,5 et 25°C. Elle est suivie jusqu' à 24 heures.
   L'extrait obtenu est très visqueux en raison de la présence de l'alginate. Cette forte viscosité rend techniquement difficile le traitement ultérieur de la solution pour extraire les fucanes. L' addition d' alginate-lyase présente un intérêt puisqu' elle permet une réduction importante de la viscosité de l'extrait. La baisse de viscosité est observée pour un rapport enzyme/algue de 1 %. On observe que la chute de viscosité de l' extrait est très rapide. Au terme de 4 heures de réaction, la viscosité correspond à environ 12 % de la valeur initiale.
   Le test est réalisé sur un viscosimètre selon un programme de mesure de la viscosité en fonction du temps à un gradient de vitesse égal à 1 350 s⁻¹.
5) Arrêt de la réaction par acidification à pH 4 (addition d'acide chlorhydrique 1 mole/l) et incubation 20 minutes à 85°C.
6) Centrifugation de l'extrait obtenu et récupération du surnageant qui contient principalement les fucanes et les alginates dépolymérisés.
7) Précipitation des alginates non dépolymérisés résiduels de l'extrait par traitement au chlorure de calcium anhydre.
8) Centrifugation de l'extrait et récupération du surnageant.
9) Purification de l'extrait par dialyse sur membrance à seuil de coupure 3 500 Dalton ou par ultrafiltration sur membrane à 30 000 Dalton.
10) Lyophilisation de l' extrait final.

Le lyophilisat final a ensuite été caractérisé par :
- le dosage des fucoses ("oses" majoritairement constitutifs des fucanes) ;
- le dosage de sulfates ;
- l' évaluation de la teneur en macromolécules en fin de purification. Elles sont essentiellement constituées de fucanes ;

La caractérisation du lyophilisat final a permis d'obtenir selon les différents essais (pourcentage d'enzyme, temps d'incubation) des rendements en macromolécules de 15 à 24 %. Les rendements obtenus sont donc meilleurs que ceux atteints par les méthodes actuelles qui sont de 2 à 3 %.

## Revendications

1. Souche bactérienne de Pseudomonas alginovora déposée le 6 mars 1998 auprès de la C.N.C.M. sous le numéro d'enregistrement 1 - 1989.

2. Alginate-lyase obtenue par la mise en culture de la souche bactérienne selon la revendication 1.

3. Utilisation de la souche selon la revendication 1 pour la production d' une alginate-lyase à activité mannuronate- et guluronate-lyase.

4. Procédé de préparation d'alginate-lyase, **caractérisé par le fait que** l'on met en culture la souche selon la revendication 1, dans un milieu qui comprend une source d'azote, une source de carbone, un extrait de levure, de l'eau de mer et un agent anti-mousse, et que l'on récupère l'alginate-lyase obtenue.

5. Procédé selon la revendication 4, **caractérisé par le fait que** la source de carbone consiste en de la protéine de pommes de terre.

6. Procédé selon la revendication 4 ou 5, **caractérisé par le fait que** la source de carbone consiste en du dextrose monohydraté.

7. Procédé selon l'une des revendications 4 à 6, **caractérisé par le fait que** l'eau de mer est filtrée à un micromètre.

8. Procédé selon l'une des revendications 4 à 7, **caractérisé par le fait que** l'agent anti-mousse est de l' huile de soja.

9. Procédé selon l'une des revendications 4 à 8, **caractérisé par le fait que** le pH du milieu est ajusté à 8,2.

10. Procédé de dépolymérisation d'alginate qui consiste à mettre de l'alginate à incuber dans une solution contenant au moins un sel mono- ou divalent à une force ionique au moins égale à 0,05 M, en présence d' une alginate-lyase produite à partir d'une souche de bactérie selon la revendication 1, et éventuellement, à récupérer la fraction de fragments d'alginate dépolymérisé obtenus.

11. Procédé selon la revendication 10, **caractérisé par le fait que** le pH de la solution est de l'ordre de 7,5.

12. Procédé selon la revendication 10 ou 11, **caractérisé par le fait que** l'alginate-lyase se présente sous forme sèche ou liquide et que dans ce dernier cas, ledit liquide consiste en l'enzyme solubilisée dans une solution tampon à pH 7,5 de trishydroxyméthylaminométhane (2-amino-2-hydroxyméthyl-1,3-propanediol) et de chlorure de sodium.

13. Procédé selon l'une des revendications 10 à 12, **caractérisé par le fait que** l'on utilise de 0,1 à 5 % en poids d'enzyme par rapport au poids d'alginate.

14. Procédé selon l'une des revendications 10 à 13, **caractérisé par le fait qu'** on laisse incuber pendant 2 à 20 heures.

15. Procédé selon la revendication 14, **caractérisé par le fait qu'**on laisse incuber pendant 8 heures environ.

16. Procédé selon l'une des revendications 10 à 15, **caractérisé par le fait que** la solution contenant au moins un sel mono- ou divalent est tamponnée, par exemple avec du trishydroxyméthylaminométhane.

17. Procédé selon l'une des revendications 10 à 16, **caractérisé par le fait que** le sel consiste en un chlorure ou en un sulfate.

18. Procédé selon la revendication 17, **caractérisé par le fait que** le chlorure consiste en un chlorure de sodium ou en un chlorure de magnésium hexahydraté.

19. Procédé selon l'une des revendications 10 à 18, **caractérisé par le fait qu'**il est mis en oeuvre dans le cadre de la production d'oligosaccharides.

20. Procédé selon l'une des revendications 10 à 19, **caractérisé par le fait qu'**il est mis en oeuvre dans le cadre de la réduction de la viscosité d'un milieu contenant de l'alginate.

21. Procédé d'alginate selon l'une des revendications 10 à 19, **caractérisé par le fait qu'**il est mis en oeuvre dans le cadre de l'extraction des fucanes d'algues brunes.

22. Fragments d'alginate dépolymérisé obtenus conformément au procédé selon l'une des revendications 10 à 21.

## Claims

1. The strain of Pseudomonas alginovora bacteria filed on March 6, 1998 with the (French) National Collection of Microorganism Cultures (C.N.C.M.) under registration No. 1-1989.

2. The alginate-lyase obtained by culturing the bacterial strain of claim 1.

3. The use of the strain of claim 1 for producing an alginate-lyase presenting mannuronate lyase and guluronate lyase activity.

4. A method of preparing alginate-lyase, **characterized by** the fact that the strain of claim 1 is cultured in a medium which comprises a source of nitrogen, a source of carbon, a yeast extract, sea water, and an anti-foaming agent, and the resulting alginate-lyase is recovered.

5. A method according to claim 4, **characterized by** the fact that the source of carbon consists in potato protein.

6. A method according to claim 4 or claim 5, **characterized by** the fact that the source of carbon consists in dextrose monohydrate.

7. A method according to any one of claims 4 to 6, **characterized by** the fact that the sea water is filtered to one micrometer.

8. A method according to any one of claims 4 to 7, **characterized by** the fact that the anti-foaming agent is soybean oil.

9. A method according to any one of claims 4 to 8, **characterized by** the fact that the pH of the medium is adjusted to 8.2.

10. A method of depolymerizing alginate, which method consists in putting the alginate for incubation in a solution containing at least one mono- or di-valent salt with ionic force of at least 0.05 M in the presence of an alginate-lyase produced from a strain of bacteria according to claim 1, and optionally in recovering the resulting fraction of depolymerized alginate fragments.

11. A method according to claim 10, **characterized by** the fact that the pH of the solution is about 7.5.

12. A method according to claim 10 or claim 11, **characterized by** the fact that the alginate-lyase is in dry or liquid form, and when in liquid form, said liquid consists in the enzyme dissolved in a buffer solution at pH 7.5 of trishydroxymethylaminomethane (2-amino-2-hydroxymethyl-1,3-propanediol) and sodium chloride.

13. A method according to any one of claims 10 to 12, **characterized by** the fact that 0.1 % to 5% by weight of the enzyme is used relative to the weight of alginate.

14. A method according to any one of claims 10 to 13, **characterized by** the fact that incubation is allowed to take place for 2 hours to 20 hours.

15. A method according to claim 14, **characterized by** the fact that incubation is allowed to take place for about 8 hours.

16. A method according to any one of claims 10 to 15, **characterized by** the fact that the solution containing at least one mono- or di-valent salt is buffered, e.g. with trishydroxymethylaminomethane.

17. A method according to any one of claims 10 to 16, **characterized by** the fact that the salt consists in a chloride or a sulfate.

18. A method according to claim 17, **characterized by** the fact that the chloride consists in sodium chloride or in magnesium chloride hexahydrate.

19. A method according to any one of claims 10 to 18, **characterized by** the fact that it is implemented in the context of producing oligosaccharides.

20. A method according to any one of claims 10 to 19, **characterized by** the fact that it is implemented in the context of reducing the viscosity of a medium containing alginate.

21. An alginate method according to any one of claims 10 to 19, **characterized by** the fact that it is implemented in the context of extracting brown seaweed fucanes.

22. Fragments of depolymerized alginate obtained in accordance with the method according to any one of claims 10 to 21.

## Patentansprüche

1. Pseudomonas alginovora Bakterienstamm, der am 6. März 1998 unter der Registrierungsnummer I - 1989 bei der CNCM hinterlegt wurde.

2. Alginat-Lyase, die durch Anlegen einer Kultur des Bakterienstamms nach Anspruch 1 gewonnen wird.

3. Verwendung des Bakterienstamms nach Anspruch 1 zur Herstellung einer Alginat-Lyase mit Mannuronat- und Guluronat-Lyase-Aktivität.

4. Verfahren zur Präparation von Alginat-Lyase, **dadurch gekennzeichnet, daß** eine Kultur des Stamms nach Anspruch 1 in einem Medium angelegt wird, das eine Stickstoffquelle, eine Kohlenstoffquelle, einen Hefe-Extrakt, Meerwasser und ein Schaumverhinderungsmittel enthält und dadurch, daß die erhaltene Alginat-Lyase zurückgewonnen wird.

5. Verfahren nach Anspruch 4, **dadurch gekennzeichnet, daß** die Kohlenstoffquelle aus Kartoffelprotein besteht.

6. Verfahren nach Anspruch 4 oder 5, **dadurch gekennzeichnet, daß** die Kohlenstoffquelle aus Dextrose-Monohydrat besteht.

7. Verfahren nach einem der Ansprüche 4 bis 6, **dadurch gekennzeichnet, daß** das Meerwasser durch einen 1 Mikrometer Filter filtriert ist.

8. Verfahren nach einem der Ansprüche 4 bis 7, **dadurch gekennzeichnet, daß** das Schaumverhinderungsmittel Sojaöl ist.

9. Verfahren nach einem der Ansprüche 4 bis 8, **dadurch gekennzeichnet, daß** der pH-Wert des Mediums auf 8,2 eingestellt ist.

10. Verfahren zur Depolymerisation von Alginat, das darin besteht, Alginat in Gegenwart einer aus einem Bakterienstamm nach Anspruch 1 gewonnenen Alginat-Lyase, in einer Lösung zu inkubieren, die wenigstens ein ein- oder zweiwertiges Salz mit einer Ionenstärke von wenigstens 0,05 M enthält, und gegebenenfalls darin, die Fraktion der gewonnenen Fragmente des depolymerisierten Alginats zurückzugewinnen.

11. Verfahren nach Anspruch 10, **dadurch gekennzeichnet, daß** der pH-Wert der Lösung bei 7,5 liegt.

12. Verfahren nach Anspruch 10 oder 11, **dadurch gekennzeichnet, daß** die Alginat-Lyase in trockener oder flüssiger Form vorliegt und daß im letzteren Fall die Flüssigkeit aus dem Enzym besteht, das in einer Pufferlösung mit pH 7,5 aus Trishydroxymethylaminomethan (2-Amino-2-hydroxymethyl-1,3-propandiol) und Natriumchlorid in Lösung gebracht wird.

13. Verfahren nach einem der Ansprüche 10 bis 12, **dadurch gekennzeichnet, daß** 0,1 bis 5 Gewichtsprozent Enzym bezogen auf das Gewicht des Alginats verwendet werden.

14. Verfahren nach einem der Ansprüche 10 bis 13, **dadurch gekennzeichnet, daß** während 2 bis 20 Stunden inkubiert wird.

15. Verfahren nach Anspruch 14, **dadurch gekennzeichnet, daß** während ungefähr 8 Stunden inkubiert wird.

16. Verfahren nach einem der Ansprüche 10 bis 15, **dadurch gekennzeichnet, daß** die Lösung, die wenigstens ein ein- oder zweiwertiges Salz enthält, beispielsweise mit Trishydroxymethylaminomethan gepuffert ist.

17. Verfahren nach einem der Ansprüche 10 bis 16, **dadurch gekennzeichnet, daß** das Salz aus einem Chlorid oder einem Sulfat besteht.

18. Verfahren nach Anspruch 17, **dadurch gekennzeichnet, daß** das Chlorid aus einem Natriumchlorid oder einem Magnesiumchlorid-Hexahydrat besteht.

19. Verfahren nach einem der Ansprüche 10 bis 18, **dadurch gekennzeichnet, daß** es im Rahmen der Herstellung von Oligosacchariden verwendet wird.

20. Verfahren nach einem der Ansprüche 10 bis 19, **dadurch gekennzeichnet, daß** es im Rahmen der Reduzierung der Viskosität eines Alginat enthaltenden Mediums verwendet wird.

21. Alginat-Verfahren nach einem der Ansprüche 10 bis 19, **dadurch gekennzeichnet, daß** es im Rahmen der Extraktion der Fucane der Braunalgen verwendet wird.

22. Fragmente depolymerisierten Alginats, die gemäß des Verfahrens nach einem der Ansprüche 10 bis 21 erhalten werden.
